# EUROPEAN PATENT SPECIFICATION

(11) **EP 3 016 084 B1**
(45) Date of publication and mention of the grant of the patent: **04.04.2018**
(21) Application number: 15191560.0
(22) Date of filing: 27.10.2015
(51) Int. Cl.: G08C 17/02

(54) **METHODS AND DEVICES FOR MODE SWITCH**
VERFAHREN UND VORRICHTUNGEN FÜR MODUSSCHALTER
PROCÉDÉS ET DISPOSITIFS POUR COMMUTATEUR DE MODE

(30) Priority: 29.10.2014 CN 201410596717
(43) Date of publication of application: 04.05.2016
(73) Proprietor: Xiaomi Inc., Beijing 100085 (CN)
(72) Inventor: SU, Jun, Beijing 100085 (CN); CHEN, Anju, Beijing 100085 (CN); WANG, Yi, Beijing 100085 (CN); WU, Yi, Beijing 100085 (CN)
(74) Representative: Delumeau, François Guy

(56) References cited:
- EP-A1- 2 263 530
- US-A1- 2012 254 909

## Description

### TECHNICAL FIELD

The present disclosure generally relates to the field of automatic control, and more particularly, to methods and devices for switching an operation mode.

### BACKGROUND

With the popularity of a wide variety of household electrical appliances, a user's sleep is often accompanied with many household electrical appliances in operation at home. For example, a fan or an air conditioner needs to be turned on in summer, a humidifier needs to be turned on when the air is dry, an air purifier needs to be turned on when air quality is poor, and so on.

In the related art, a silent mode is typically provided in a household electrical appliance such as a fan, an air conditioner, a humidifier, an air purifier or the like. When a user needs to go to sleep, he/she may adjust an operation mode of the household electrical appliance to the silent mode through a remote controller or in a manual manner. After the silent mode is enabled for the household electrical appliance, noise generated by the operation of the appliance will be substantially reduced.

Document US 2012/254909 discloses a method of operating a set-top box device to present audio/video content to the user. The method collects sensor data at the set-top box, determines from the sensor data that the user is likely to be asleep.

Document EP 2263530 discloses a method for controlling the power consumption of an arrangement of devices in dependency to the sleep status of a person.

During the implementation of the present disclosure, at least the following defects are found in the related art:
a process of adjusting the household electrical appliance to the silent mode through a remote controller or in a manual manner is still a manual control which involves troublesome operations.

### SUMMARY

Methods and devices for switching an operation mode are provided by embodiments of the present disclosure. The technical solutions are as follows.

According to a first aspect, the invention relates to a method for switching an operation mode which is applied in a household electrical appliance. The method comprises:
detecting whether a user is in a sleeping state and if the user is in the sleeping state :
   - detecting sleep quality of the user according to the physiological data collected by a wearable device; and
   - adjusting a speed of the fan in the household electrical appliance according to the sleep quality of the user and current air quality; and
the step of detecting whether a user is in a sleeping state comprises acquiring an operation state of a wireless access point, and detecting whether the user is in the sleeping state according to the operation state of the wireless access point.

According to another aspect, the invention relates to a device for switching an operation mode, which is applied in a household electrical appliance. The device comprises:
a first detecting module, configured to detect whether a user is in a sleeping state;
a first detecting submodule, configured to acquire physiological data of the user which is collected by a wearable device, and detect whether the user is in the sleeping state according to the physiological data;
a detecting module, configured to, if the household electrical appliance is provided with a fan, detect sleep quality of the user according to the physiological data collected by the wearable device;
an instruction generating module, configured to generate a speed adjusting instruction according to the sleep quality of the user; and
a sending module, configured to send the speed adjusting instruction to the household electrical appliance; the household electrical appliance being configured to adjust a speed of the fan in the household electrical appliance according to the speed adjusting instruction and current air quality; and
the detecting module comprises: a detecting submodule (12013), configured to acquire an operation state of a wireless access point, and detect whether the user is in the sleeping state according to the operation state of the wireless access point.

In a particular embodiment, the device further comprises:
a binding establishing module, configured to establish in advance a binding relationship with the wearable device and the household electrical appliance.

According to a fifth aspect, the invention relates to a device for switching an operation mode, which is applied in a household electrical appliance. The device comprises:
a processor; and
a memory for storing instructions executable by the processor;
wherein the processor is configured to perform:
detecting whether a user is in a sleeping state and if the user is in the sleeping state:
   - detecting sleep quality of the user according to physiological data collected by a wearable device; the device being characterized in that the processor is configured to perform:
   - adjusting a speed of a fan in said household electrical appliance according to the sleep quality of the user and current air quality; and
the detecting whether a user is in a sleeping state comprises acquiring an operation state of a wireless access point, and detecting whether the user is in the sleeping state according to the operation state of the wireless access point.

In one particular embodiment, the steps of the method for switching an operation mode are determined by computer program instructions.

Consequently, according to a seventh aspect, the invention is also directed to a computer program for executing the steps of a method for switching an operation mode when this program is executed by a computer.

This program can use any programming language and take the form of source code, object code or a code intermediate between source code and object code, such as a partially compiled form, or any other desirable form.

The invention is also directed to a computer-readable information medium containing instructions of a computer program as described above.

The information medium can be any entity or device capable of storing the program. For example, the support can include storage means such as a ROM, for example a CD ROM or a microelectronic circuit ROM, or magnetic storage means, for example a diskette (floppy disk) or a hard disk.

Alternatively, the information medium can be an integrated circuit in which the program is incorporated, the circuit being adapted to execute the method in question or to be used in its execution.

The advantageous effects brought by the technical solutions according to the embodiments of the present disclosure include the following.

It is detected whether a user is in a sleeping state; if the user is in the sleeping state, a current operation mode is switched to a silent mode; and then the silent mode is operated. Accordingly, it may be solved the problem that a process of adjusting the household electrical appliance to the silent mode through a remote controller or in a manual manner is still a manual control which involves troublesome operations, and it may be achieved an effect that the process for switching an operation mode may be simplified, and the silent mode may be operated through automatic control.

It is to be understood that both the foregoing general description and the following detailed description are exemplary and explanatory only and are not restrictive of the invention.

### BRIEF DESCRIPTION OF THE DRAWINGS

The accompanying drawings, which are incorporated in and constitute a part of this specification, illustrate embodiments consistent with the invention and, together
a first sending module, configured to, if the user is in the sleeping state, send a mode switching instruction to the household electrical appliance; the household electrical appliance being configured to switch a current operation mode to a silent mode according to the mode switching instruction, and operate in the silent mode.

In a particular embodiment, the detecting module comprises:
a detecting submodule, configured to acquire physiological data of the user which is collected by a wearable device, and detect whether the user is in the sleeping state according to the physiological data;
   and/or,
a detecting submodule, configured to acquire an operation state of a wireless access point, and detect whether the user is in the sleeping state according to the operation state of the wireless access point;
   and/or,
a detecting submodule, configured to acquire a noise value of a current environment, and detect whether the user is in the sleeping state according to the noise value of the current environment.

In a particular embodiment, the device further comprises:
a detecting module, configured to, if the household electrical appliance is provided with a fan, detect sleep quality of the user according to the physiological data collected by the wearable device;
an instruction generating module, configured to generate a speed adjusting instruction according to the sleep quality of the user; and
a second sending module, configured to send the speed adjusting instruction to the household electrical appliance; the household electrical appliance being configured to adjust a speed of the fan in the household electrical appliance according to the speed adjusting instruction.

In a particular embodiment, the instruction generating module is configured to generate the speed adjusting instruction according to the sleep quality of the user and current air quality.

In a particular embodiment, the device further comprises:
a binding establishing module, configured to establish in advance a binding relationship with the wearable device and the household electrical appliance.

According to a fifth aspect, the invention relates to a device for switching an operation mode, which is applied in a household electrical appliance. The device comprises:
a processor; and
a memory for storing instructions executable by the processor;
wherein the processor is configured to perform:
detecting whether a user is in a sleeping state;
if the user is in the sleeping state, switching a current operation mode to a silent mode; and
operating in the silent mode.

According to a sixth aspect, the invention relates to a device for switching an operation mode, which is applied in a controlling device coupled to a household electrical appliance. The device comprises:
a processor; and
a memory for storing instructions executable by the processor;
wherein the processor is configured to perform:
detecting whether a user is in a sleeping state; and
if the user is in the sleeping state, sending a mode switching instruction to the household electrical appliance; the household electrical appliance being configured to switch a current operation mode to a silent mode according to the mode switching instruction, and operate in the silent mode.

In one particular embodiment, the steps of the method for switching an operation mode are determined by computer program instructions.

Consequently, according to a seventh aspect, the invention is also directed to a computer program for executing the steps of a method for switching an operation mode when this program is executed by a computer.

This program can use any programming language and take the form of source code, object code or a code intermediate between source code and object code, such as a partially compiled form, or any other desirable form.

The invention is also directed to a computer-readable information medium containing instructions of a computer program as described above.

The information medium can be any entity or device capable of storing the program. For example, the support can include storage means such as a ROM, for example a CD ROM or a microelectronic circuit ROM, or magnetic storage means, for example a diskette (floppy disk) or a hard disk.

Alternatively, the information medium can be an integrated circuit in which the program is incorporated, the circuit being adapted to execute the method in question or to be used in its execution.

The advantageous effects brought by the technical solutions according to the embodiments of the present disclosure include the following.

It is detected whether a user is in a sleeping state; if the user is in the sleeping state, a current operation mode is switched to a silent mode; and then the silent mode is operated. Accordingly, it may be solved the problem that a process of adjusting the household electrical appliance to the silent mode through a remote controller or in a manual manner is still a manual control which involves troublesome operations, and it may be achieved an effect that the process for switching an operation mode may be simplified, and the silent mode may be operated through automatic control.

It is to be understood that both the foregoing general description and the following detailed description are exemplary and explanatory only and are not restrictive of the invention.

### BRIEF DESCRIPTION OF THE DRAWINGS

The accompanying drawings, which are incorporated in and constitute a part of this specification, illustrate embodiments consistent with the invention and, together with the description, serve to explain the principles of the invention.
Fig.1 is a schematic diagram of an implementing environment involved in a method for switching an operation mode provided by an embodiment of the present disclosure;
Fig.2 is a flow chart of a method for switching an operation mode according to an exemplary embodiment;
Fig.3 is a flow chart of a method for switching an operation mode according to another exemplary embodiment;
Fig.4 is a schematic diagram of an implementing environment involved in a method for switching an operation mode provided by another embodiment of the present disclosure;
Fig.5 is a flow chart of a method for switching an operation mode according to still another exemplary embodiment;
Fig.6 is a flow chart of a method for switching an operation mode according to further still another exemplary embodiment;
Fig.7 is a schematic diagram of an implementing environment involved in a method for switching an operation mode provided by still another embodiment of the present disclosure;
Fig.8 is a flow chart of a method for switching an operation mode according to further still another exemplary embodiment;
Fig.9 is a block diagram of a device for switching an operation mode according to an exemplary embodiment;
Fig.10 is a block diagram of a device for switching an operation mode according to another exemplary embodiment;
Fig.11 is a block diagram of a device for switching an operation mode according to still another exemplary embodiment;
Fig.12 is a block diagram of a device for switching an operation mode according to further still another exemplary embodiment;

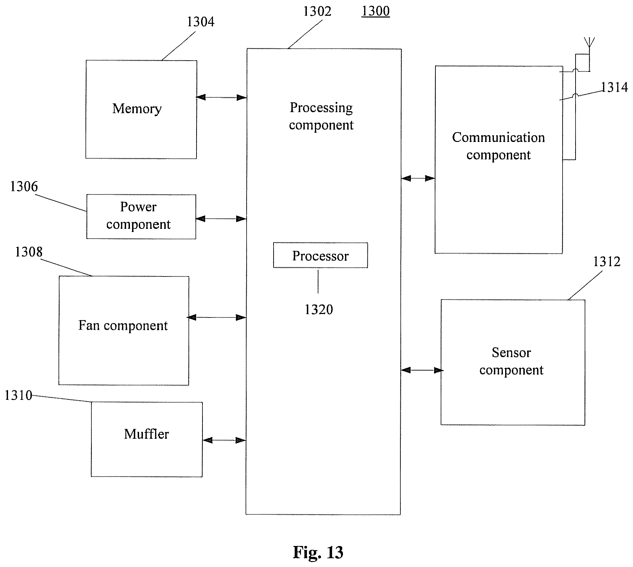

Fig.13 is a block diagram of a device for switching an operation mode according to an exemplary embodiment; and
Fig.14 is a block diagram of a device for switching an operation mode according to another exemplary embodiment.

Through the above accompany drawings, the specific embodiments of the disclosure have been illustrated, for which a more detailed description will be given hereinafter. These drawings and textual description are not intended to limit the scope of the concept of the disclosure in any manner, but to explain the concept of the disclosure to those skilled in the art through particular embodiments.

### DETAILED DESCRIPTION

Reference will now be made in detail to exemplary embodiments, examples of which are illustrated in the accompanying drawings. The following description refers to the accompanying drawings in which the same numbers in different drawings represent the same or similar elements unless otherwise represented. The implementations set forth in the following description of exemplary embodiments do not represent all implementations consistent with the invention. Instead, they are merely examples of devices and methods consistent with aspects related to the invention as recited in the appended claims.

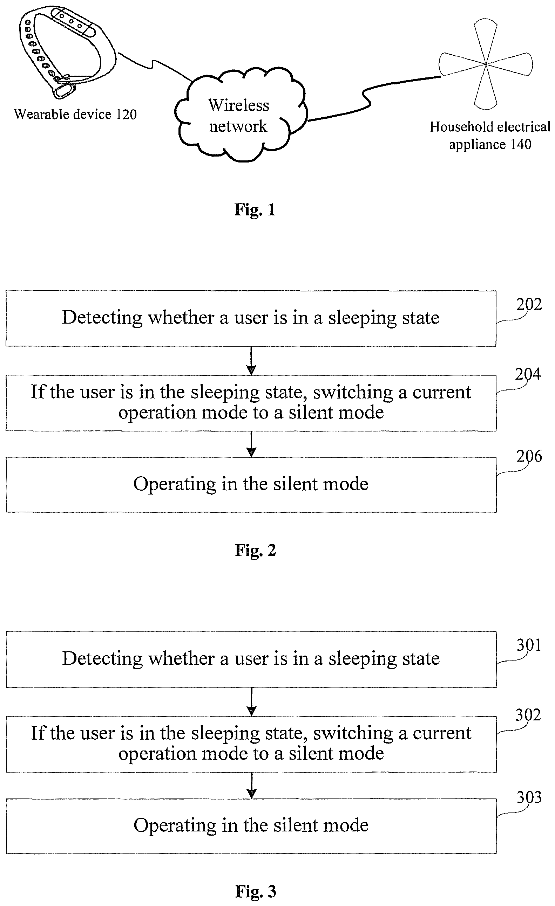

Referring to Fig.1, which is a schematic diagram of an implementing environment involved in a method for switching an operation mode provided by an embodiment of the present disclosure. The implementing environment includes: a wearable device 120 and a household electrical appliance 140.

Wherein, the wearable device 120 may be connected to the household electrical appliance 140 through a wireless connection. The wireless connection may be a Bluetooth connection or a WiFi (Wireless-Fidelity) connection.

The wearable device 120 is an electronic device which is capable of acquiring physiological data of the user, and sending the physiological data to the household electrical appliance 140. The physiological data may include at least one of heartbeat, pulse and body movement. In practical implementation, the wearable device 120 may be a smart wristband, a smart watch, a smart bracelet, a smart necklace, a smart ring or a smart glass. The wearable device 120 is provided with a sensor configured to acquire the physiological data of the user, and the sensor may be a pulse sensor, a heart rate sensor, a gravity sensor and a gyro sensor, etc.

The household electrical appliance 140 may include at least one of a fan, an air conditioner, a humidifier and an air purifier. In the embodiments of the present disclosure, the household electrical appliance is for example an air purifier.

Fig.2 is a flow chart of a method for switching an operation mode according to an exemplary embodiment. In the present embodiment, the method for switching an operation mode is described as being applied, for example, in the implementation environment as shown in Fig.1. As shown in Fig.2, the method for switching an operation mode may include the following steps.

In step 202, it is detected whether a user is in a sleeping state.

In step 204, if the user is in the sleeping state, a current operation mode is switched to a silent mode.

In step 206, it is operated in the silent mode.

In the method for switching an operation mode provided by the present embodiment, it is detected whether a user is in a sleeping state; if the user is in the sleeping state, a current operation mode is switched to a silent mode; and then it is operated in the silent mode. Accordingly, it may be solved the problem that a process of adjusting the household electrical appliance to the silent mode through a remote controller or in a manual manner is still a manual control which involves troublesome operations, and it may be achieved an effect that the process for switching an operation mode may be simplified, and the silent mode may be operated through automatic control.

Fig.3 is a flow chart of a method for switching an operation mode according to another exemplary embodiment. In the present embodiment, the method for switching an operation mode is described as being applied, for example, in the implementation environment as shown in Fig.1. The method for switching an operation mode may include the following steps.

In step 301, it is detected whether a user is in a sleeping state.

The household electrical appliance detects whether the user is in the sleeping state, which may include three different implementations, respectively as follows.

As a first possible implementation, the step 301 may include but not limited to the following substeps.
1. The wearable device collects physiological data of the user, and the physiological data may include at least one of heartbeat, pulse and body movement.
2. The wearable device sends the collected physiological data of the user to the household electrical appliance.
3. The household electrical appliance detects whether the user is in the sleeping state according to the physiological data.

When the heartbeat and pulse collected by the wearable device is below a normal value for a non-sleeping state and/or the corresponding body movement is below a predetermined frequency, the detection result of the household electrical appliance is the user is in the sleeping state. When the heartbeat and pulse collected by the wearable device is at the normal value for a non-sleeping state and/or the corresponding body movement is above the predetermined frequency, the detection result of the household electrical appliance is the user is in a non-sleeping state.

As a second possible implementation, the step 301 may include but not limited to the following substeps.
1. The household electrical appliance acquires an operation state of a wireless access point.

The wireless access point may be a router supporting a Wi-Fi network at home. The wireless access point may be turned on or turned off manually to change the operation state of the wireless access point, or may be turned on during running time preset by the user, and turned off in non-running time, thereby to change the operation state of the wireless access point.

For example, the user presets 9:00-23:00 as the running time of the wireless access point, and 23:00-9:00 the next day as the non-running time of the wireless access point. When it is 9:00-23:00, the household electrical appliance acquires that the wireless access point is in the running state, and when it is 23:00-9:00 the next day, the household electrical appliance acquires that the wireless access point is in the non-running state.
2. The household electrical appliance detects whether the user is in the sleeping state according to the operation state of the wireless access point.

When the wireless access point is in the running state, the detection result of the household electrical appliance is the user is in the non-sleeping state; and when the wireless access point is in the non-running state, the detection result of the household electrical appliance is the user is in the sleeping state.

As a third possible implementation, the step 301 may include but not limited to the following substeps.
1. The household electrical appliance acquires a noise value of a current environment.

The household electrical appliance acquires the noise value of the current environment through a built-in noise meter or microphone.
2. The household electrical appliance detects whether the user is in the sleeping state according to the noise value of the current environment.

When the noise value of the current environment is larger than a bearable noise value for the sleeping state, the detection result of the household electrical appliance is the user is in the non-sleeping state; and when the noise value of the current environment is smaller than the bearable noise value for the sleeping state, the detection result of the household electrical appliance is the user is in the sleeping state.

It should be noted that, in practical implementation of the present step, whether the user is in the sleeping state may be detected through one of the implementations; or whether the user is in the sleeping state may be detected through combination of any two of the implementations; or whether the user is in the sleeping state may be detected through combination of the three implementations. Wherein, reliability of the detection result through the combination of the three implementations is higher than reliability of the detection result through the combination of any two of the implementations, and the reliability of the detection result through the combination of any two of the implementations is higher than reliability of the detection result through one implementation.

In the combination of any two of the implementations or the combination of the three implementations, a weight may be assigned to each implementation of the two or three implementations according to a certain proportion of weights, and the detection result of the combined implementation may be calculated by taking all the weights into consideration.

In step 302, if the user is in the sleeping state, a current operation mode is switched to a silent mode.

In step 303, it is operated in the silent mode.

The household electrical appliance operates in the silent mode. Operating in the silent mode involves for example the following situations.

When the household electrical appliance is provided with a muffler, for example it is an air purifier provided with a muffler, the household electrical appliance enables the muffler. The muffler may be a muffler layer or a muffler component disposed in an air flow passage or in an air intake system or an air exhaust system of an aerodynamic device (a device with a loud noise at an exhaust port, such as a fan or a blower).

When the household electrical appliance is provided with a fan, sleep quality of the user is detected according to the physiological data collected by the wearable device; and a speed of the fan in the household electrical appliance is adjusted according to the sleep quality of the user.

The substep 2 may include but not limited to the following substeps.
1. The household electrical appliance detects the sleep quality of the user according to the physiological data collected by the wearable device.

When the user is in the sleep state, the sleep quality of the user may be divided into first sleep quality and second sleep quality. The first sleep quality may also be referred to as a "light sleep state", the second sleep quality may also be referred to as a "deep sleep state", and the sleep effect of the first sleep quality is poorer than that of the second sleep quality.

If the physiological data is consistent with physiological data of the first sleep quality, the detection result of the household electrical appliance is the sleep quality of the user is in the first sleep quality. If the physiological data is consistent with physiological data of the second sleep quality, the detection result of the household electrical appliance is the sleep quality of the user is in the second sleep quality.
2. The speed of the fan in the household electrical appliance is adjusted according to the sleep quality of the user.

When the sleep quality of the user is in the first sleep quality, the household electrical appliance adjusts the speed of the fan to a first speed; and when the sleep quality of the user is in the second sleep quality, the household electrical appliance adjusts the speed of the fan to a second speed. Wherein, since the sleep effect of the first sleep quality is poorer than that of the second sleep quality, the first speed is smaller than the second speed.

For example, Table 1 shows an example for adjusting the speed of the fan according to the sleep quality.

| sleep quality | first sleep quality | second sleep quality |
|---|---|---|
| speed of the fan | A r/s | B r/s |

Wherein, A<B.

The substep 2 may further include another implementation.

The household electrical appliance adjusts the speed of the fan according to the sleep quality of the user and current air quality. That is, the household electrical appliance may take both of the sleep quality of the user and the current air quality into consideration. The speed of the fan after the adjustment positively correlates to the sleep quality, and the speed of the fan after the adjustment negatively correlates to the current air quality.

Assuming that the current air quality may be divided into first air quality, second air quality and third air quality. Wherein, the first air quality is better than the second air quality, and the second air quality is better than the third air quality.

When the sleep quality of the user is in the first sleep quality, and the current air quality is in the first air quality, the household electrical appliance adjusts the speed of the fan to the first speed;
when the sleep quality of the user is in the second sleep quality, and the current air quality is in the first air quality, the household electrical appliance adjusts the speed of the fan to the second speed;
when the sleep quality of the user is in the first sleep quality, and the current air quality is in the second air quality, the household electrical appliance adjusts the speed of the fan to the third speed;
when the sleep quality of the user is in the second sleep quality, and the current air quality is in the second air quality, the household electrical appliance adjusts the speed of the fan to the fourth speed;
when the sleep quality of the user is in the first sleep quality, and the current air quality is in the third air quality, the household electrical appliance adjusts the speed of the fan to the fifth speed; and
when the sleep quality of the user is in the second sleep quality, and the current air quality is in the third air quality, the household electrical appliance adjusts the speed of the fan to the sixth speed.

Wherein, since the sleep effect of the first sleep quality is poorer than that of the second sleep quality, the first air quality is better than the second air quality, and the second air quality is better than the third air quality, then, the first speed is smaller than the second speed, the second speed is smaller than the third speed, the third speed is smaller than the fourth speed, the fourth speed is smaller than the fifth speed, and the fifth speed is smaller than the sixth speed.

For example, Table 2 shows an example for adjusting the speed of the fan according to the sleep quality and the current air quality.

| speed | first sleep quality (light sleep) | second sleep quality (deep sleep) |
|---|---|---|
| first air quality | A r/s | B r/s |
| second air quality | C r/s | D r/s |
| third air quality | E r/s | F r/s |

Wherein, A<B<C<D<E<F.

In the method for switching an operation mode provided by the present embodiment, it is detected whether a user is in a sleeping state; if the user is in the sleeping state, a current operation mode is switched to a silent mode; and then it is operated in the silent mode. Accordingly, it may be solved the problem that a process of adjusting the household electrical appliance to the silent mode through a remote controller or in a manual manner is still a manual control which involves troublesome operations, and it may be achieved an effect that the process for switching an operation mode may be simplified, and the silent mode may be operated through automatic control.

In addition, in the method for switching an operation mode provided by the present embodiment, the sleep quality of the user is detected accorded to the physiological data collected by the wearable device in the silent mode; and the speed of the fan in the household electrical appliance is adjusted according to the sleep quality of the user. Accordingly, it may be achieved an effect that the speed of the fan may be automatically adjusted according to the sleep quality and the noise may be reduced.

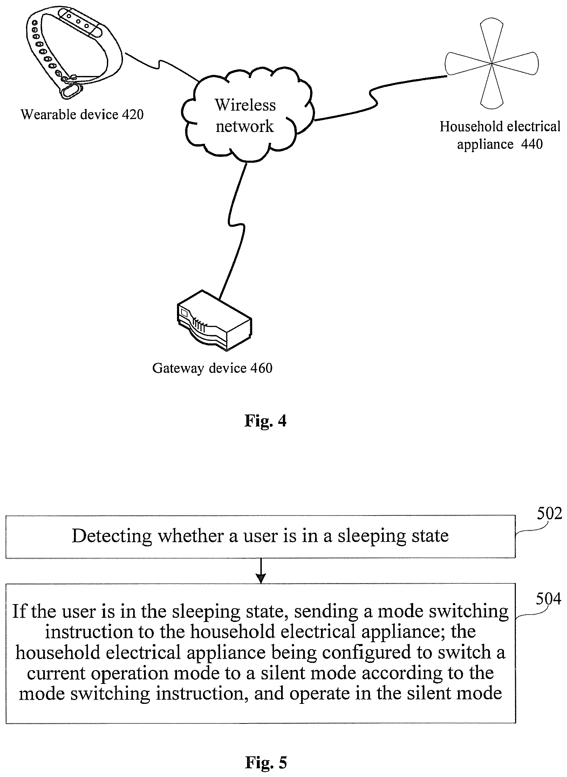

Referring to Fig.4, which is a schematic diagram of an implementing environment involved in a method for switching an operation mode provided by another embodiment of the present disclosure. The implementing environment includes: a wearable device 420, a household electrical appliance 440 and a gateway device 460.

Wherein, the wearable device 420 and the household electrical appliance 440 may be connected to the gateway device 460 through a wireless connection. The wireless connection may be a Bluetooth connection or a WiFi connection.

The wearable device 420 is an electronic device which is capable of acquiring physiological data of the user, and sending the physiological data to the gateway device 460. The physiological data may include at least one of heartbeat, pulse and body movement. In practical implementation, the wearable device 420 may be a smart wristband, a smart watch, a smart bracelet, a smart necklace, a smart ring or a smart glass. The wearable device 420 is provided with a sensor configured to acquire the physiological data of the user, and the sensor may be a pulse sensor, a heart rate sensor, a gravity sensor and a gyro sensor, etc.

The household electrical appliance 440 may include at least one of a fan, an air conditioner, a humidifier and an air purifier. In the embodiments of the present disclosure, the household electrical appliance is for example an air purifier.

The gateway device 460 may be a router or an exchanger. In the present embodiment, the gateway device is for example a router.

Fig.5 is a flow chart of a method for switching an operation mode according to still another exemplary embodiment. In the present embodiment, the method for switching an operation mode is described as being applied, for example, to the gateway device 460 as shown in Fig.4. As shown in Fig.5, the method for switching an operation mode may include the following steps.

In step 502, it is detected whether a user is in a sleeping state.

In step 504, if the user is in the sleeping state, a mode switching instruction is sent to the household electrical appliance; the household electrical appliance being configured to switch a current operation mode to a silent mode according to the mode switching instruction, and operate in the silent mode.

In the method for switching an operation mode provided by the present embodiment, the gateway device detects whether a user is in a sleeping state; and if the user is in the sleeping state, a mode switching instruction is sent to the household electrical appliance; the household electrical appliance being configured to switch a current operation mode to a silent mode according to the mode switching instruction, and operate in the silent mode. Accordingly, it may be solved the problem that a process of adjusting the household electrical appliance to the silent mode through a remote controller or in a manual manner is still a manual control which involves troublesome operations, and it may be achieved an effect that the process for switching an operation mode may be simplified, and the silent mode may be operated through automatic control.

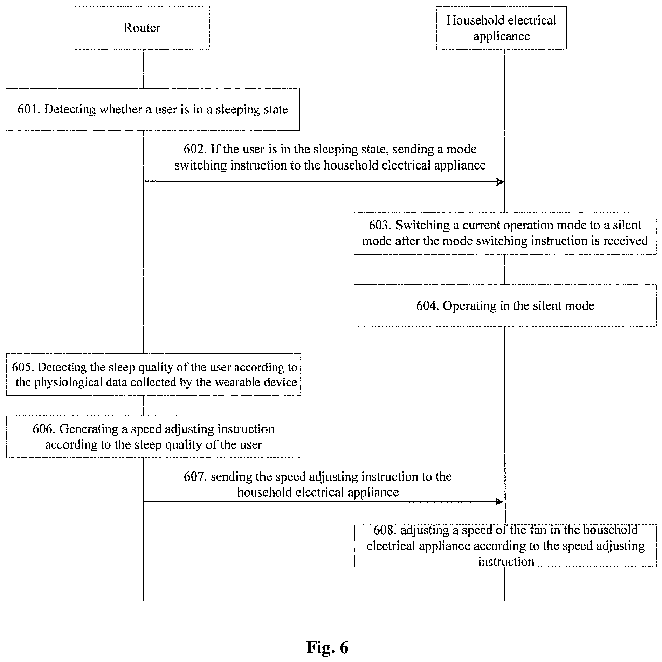

Fig.6 is a flow chart of a method for switching an operation mode according to further still another exemplary embodiment. In the present embodiment, the method for switching an operation mode is described as being applied, for example, in the implementation environment as shown in Fig.4. The method for switching an operation mode may include the following steps.

In step 601, the router detects whether a user is in a sleeping state.

The router detects whether the user is in the sleeping state, which may include three different implementations, respectively as follows.

As a first possible implementation, the step 601 may include but not limited to the following substeps.
1. The wearable device collects physiological data of the user, and the physiological data may include at least one of heartbeat, pulse and body movement.
2. The wearable device sends the collected physiological data of the user to the router.
3. The router detects whether the user is in the sleeping state according to the physiological data.

When the heartbeat and pulse collected by the wearable device is below a normal value for a non-sleeping state and/or the corresponding body movement is below a predetermined frequency, the detection result of the router is the user is in the sleeping state. When the heartbeat and pulse collected by the wearable device is at the normal value for a non-sleeping state and/or the corresponding body movement is above the predetermined frequency, the detection result of the router is the user is in a non-sleeping state.

As a second possible implementation, the step 601 may include but not limited to the following substeps.
1. The router acquires an operation state of a wireless access point.

The wireless access point may be a router supporting a Wi-Fi network at home. The wireless access point may be turned on or turned off manually to change the operation state of the wireless access point, or may be turned on during running time preset by the user, and turned off in non-running time, thereby to change the operation state of the wireless access point.

For example, the user presets 9:00-23:00 as the running time of the wireless access point, and 23:00-9:00 the next day as the non-running time of the wireless access point. When it is 9:00-23:00, the router acquires that the wireless access point is in the running state, and when it is 23:00-9:00 the next day, the router acquires that the wireless access point is in the non-running state.
2. The router detects whether the user is in the sleeping state according to the operation state of the wireless access point.

When the wireless access point is in the running state, the detection result of the router is the user is in the non-sleeping state; and when the wireless access point is in the non-running state, the detection result of the router is the user is in the sleeping state.

As a third possible implementation, the step 601 may include but not limited to the following substeps.
1. The household electrical appliance acquires a noise value of a current environment, and sends the noise value of the current environment to the router.

The household electrical appliance acquires the noise value of the current environment through a built-in noise meter or microphone, and sends the noise value of the current environment to the router.
2. The router detects whether the user is in the sleeping state according to the noise value of the current environment.

When the noise value of the current environment is larger than a bearable noise value for the sleeping state, the detection result of the router is the user is in the non-sleeping state; and when noise value of the current environment is smaller than the bearable noise value for the sleeping state, the detection result of the router is the user is in the sleeping state.

It should be noted that, in practical implementation of the present step, whether the user is in the sleeping state may be detected through one of the implementations; or whether the user is in the sleeping state may be detected through combination of any two of the implementations; or whether the user is in the sleeping state may be detected through combination of the three implementations. Wherein, reliability of the detection result through the combination of the three implementations is higher than reliability of the detection result through the combination of any two of the implementations, and the reliability of the detection result through the combination of any two of the implementations is higher than reliability of the detection result through one implementation.

It should be further noted that, in the combination of any two of the implementations or the combination of the three implementations, a weight may be assigned to each implementation of the two or three implementations according to a certain proportion of weights, and the detection result of the combined implementation may be calculated by taking all the weights into consideration.

In step 602, if the user is in the sleeping state, the router sends a mode switching instruction to the household electrical appliance.

In step 603, the household electrical appliance switches a current operation mode to a silent mode after it receives the mode switching instruction.

In step 604, the household electrical appliance operates in the silent mode.

When the household electrical appliance is provided with a muffler, for example it is an air purifier provided with a muffler, the air purifier enables the muffler. The muffler may be a muffler layer or a muffler component for reducing noise which is disposed in an air flow passage or in an air intake system or an air exhaust system of an aerodynamic device (a device with a loud noise at an exhaust port, such as a fan or a blower).

When the household electrical appliance is further provided with a fan, the method may optionally include the following steps.

In step 605, the router detects the sleep quality of the user according to the physiological data collected by the wearable device.

When the user is in the sleep state, the sleep quality of the user may be divided into first sleep quality and second sleep quality. The first sleep quality may also be referred to as a "light sleep state", the second sleep quality may also be referred to as a "deep sleep state", and the sleep effect of the first sleep quality is poorer than that of the second sleep quality.

If the physiological data is consistent with physiological data of the first sleep quality, the detection result of the router is the sleep quality of the user is in the first sleep quality. If the physiological data is consistent with physiological data of the second sleep quality, the detection result of the router is the sleep quality of the user is in the second sleep quality.

In step 606, the router generates a speed adjusting instruction according to the sleep quality of the user.

When the sleep quality of the user is in the first sleep quality, the router generates a first speed adjusting instruction; and when the sleep quality of the user is in the second sleep quality, the router generates a second speed adjusting instruction. Wherein, since the sleep effect of the first sleep quality is poorer than that of the second sleep quality, the first speed is smaller than the second speed.

In step 607, the router sends the speed adjusting instruction to the household electrical appliance.

The router sends the speed adjusting instruction to the household electrical appliance. In the present embodiment, for example, the first sleep quality corresponds to the first speed, and the second sleep quality corresponds to the second speed. The speed adjusting instruction may be the first speed adjusting instruction, or may also be the second speed adjusting instruction. In practical implementation, the speed adjusting instruction may include at least two types of speed adjusting instructions. The speed for the speed adjusting instruction may be preset by the user, or may be preset by maintenance personnel of the router.

In step 608, the household electrical appliance adjusts a speed of the fan in the household electrical appliance according to the speed adjusting instruction.

When the first speed adjusting instruction is received, the household electrical appliance adjusts the speed of the fan to the first speed; and when the second speed adjusting instruction is received, the household electrical appliance adjusts the speed of the fan to the second speed. Wherein, since the sleep effect of the first sleep quality is poorer than that of the second sleep quality, the first speed is smaller than the second speed.

The above steps 607 and 608 have another alternative implementation.

The router adjusts the speed of the fan in the household electrical appliance according to the sleep quality of the user and current air quality.

The router detects the sleep quality of the user according to the physiological data collected by the wearable device; the household electrical appliance detects the current air quality, and sends the current air quality to the router; the router generates the speed adjusting instruction according to the sleep quality of the user and the current air quality; the router sends the speed adjusting instruction to the household electrical appliance; and the household electrical appliance adjusts the speed of the fan in the household electrical appliance according to the speed adjusting instruction.

That is, the router may take both of the sleep quality of the user and the current air quality into consideration to control the speed of the fan. The speed of the fan after the adjustment positively correlates to the sleep quality, and the speed of the fan after the adjustment negatively correlates to the current air quality.

Assuming that the current air quality may be divided into first air quality, second air quality and third air quality. Wherein, the first air quality is better than the second air quality, and the second air quality is better than the third air quality.

When the sleep quality of the user is in the first sleep quality, and the current air quality is in the first air quality, the router sends the first speed adjusting instruction to the household electrical appliance, and the household electrical appliance adjusts the speed of the fan to the first speed according to the first speed adjusting instruction;
when the sleep quality of the user is in the second sleep quality, and the current air quality is in the first air quality, the router sends the second speed adjusting instruction to the household electrical appliance, and the household electrical appliance adjusts the speed of the fan to the second speed according to the second speed adjusting instruction;
when the sleep quality of the user is in the first sleep quality, and the current air quality is in the second air quality, the router sends the third speed adjusting instruction to the household electrical appliance, and the household electrical appliance adjusts the speed of the fan to the third speed according to the third speed adjusting instruction;
when the sleep quality of the user is in the second sleep quality, and the current air quality is in the second air quality, the router sends the fourth speed adjusting instruction to the household electrical appliance, and the household electrical appliance adjusts the speed of the fan to the fourth speed according to the fourth speed adjusting instruction;
when the sleep quality of the user is in the first sleep quality, and the current air quality is in the third air quality, the router sends the fifth speed adjusting instruction to the household electrical appliance, and the household electrical appliance adjusts the speed of the fan to the fifth speed according to the fifth speed adjusting instruction; and
when the sleep quality of the user is in the second sleep quality, and the current air quality is in the third air quality, the router sends the sixth speed adjusting instruction to the household electrical appliance, and the household electrical appliance adjusts the speed of the fan to the sixth speed according to the sixth speed adjusting instruction.

Wherein, since the sleep effect of the first sleep quality is poorer than that of the second sleep quality, the first air quality is better than the second air quality, and the second air quality is better than the third air quality, then, the first speed is smaller than the second speed, the second speed is smaller than the third speed, the third speed is smaller than the fourth speed, the fourth speed is smaller than the fifth speed, and the fifth speed is smaller than the sixth speed.

It should be further noted that, the method for switching an operation mode provided by the present embodiment may also include:
the router establishes in advance a binding relationship with the wearable device and the household electrical appliance.

The router establishes in advance a binding relationship among the router, the wearable device and the household electrical appliance according to an identification of the router, an identification of the wearable device and an identification of the household electrical appliance. The household electrical appliance only receives controlling instruction sent by the router which has an established binding relationship therewith.

In the method for switching an operation mode provided by the present embodiment, it is detected whether a user is in a sleeping state; if the user is in the sleeping state, a mode switching instruction is sent to the household electrical appliance; the household electrical appliance being configured to switch a current operation mode to a silent mode, and operate in the silent mode. Accordingly, it may be solved the problem that a process of adjusting the router to the silent mode through a remote controller or in a manual manner is still a manual control which involves troublesome operations, and it may be achieved an effect that the process for switching an operation mode may be simplified, and the silent mode may be operated through automatic control.

In addition, in the method for switching an operation mode provided by the present embodiment, the sleep quality of the user is detected according to the physiological data collected by the wearable device in the silent mode; and the speed of the fan in the household electrical appliance is adjusted according to the sleep quality of the user. Accordingly, it may be achieved an effect that the speed of the fan may be automatically adjusted according to the sleep quality and the noise may be reduced.

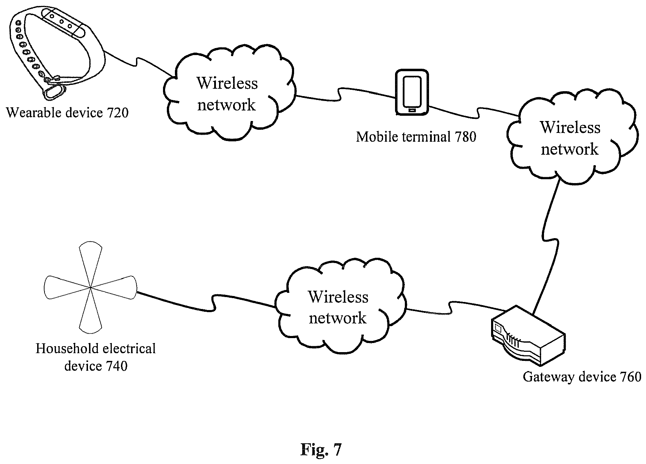

Referring to Fig.7, which is a schematic diagram of an implementing environment involved in a method for switching an operation mode provided by still another embodiment of the present disclosure. The implementing environment includes: a wearable device 720, a household electrical appliance 740, a gateway device 760 and a mobile terminal 780.

Wherein, the mobile terminal 780 and the household electrical appliance 740 are respectively connected to the gateway device 760 through a wireless connection. The wireless connection may be a Bluetooth connection or a WiFi connection. The wearable device 720 may also be connected to the mobile terminal 780 through a wireless connection.

The wearable device 720 is an electronic device which is capable of acquiring physiological data of the user, and sending the physiological data to the gateway device 760. The physiological data may include at least one of heartbeat, pulse and body movement. In practical implementation, the wearable device 720 may be a smart wristband, a smart watch, a smart bracelet, a smart necklace, a smart ring or a smart glass. The wearable device 720 is provided with a sensor configured to acquire the physiological data of the user, and the sensor may be a pulse sensor, a heart rate sensor, a gravity sensor and a gyro sensor, etc.

The household electrical appliance 740 may include at least one of a fan, an air conditioner, a humidifier and an air purifier. In the embodiments of the present disclosure, the household electrical appliance is for example an air purifier.

The gateway device 760 may be a router or an exchanger. In the present embodiment, the gateway device is for example a router.

The mobile terminal 780 may be an electronic device such as a smart phone, a tablet computer, an electronic reader, a notebook computer or the like. The mobile terminal 780 functions similarly to the gateway device 460 as shown in Fig.4. Both the mobile terminal 780 and the gateway device 460 may be referred to as a controlling device coupled to a household electrical appliance.

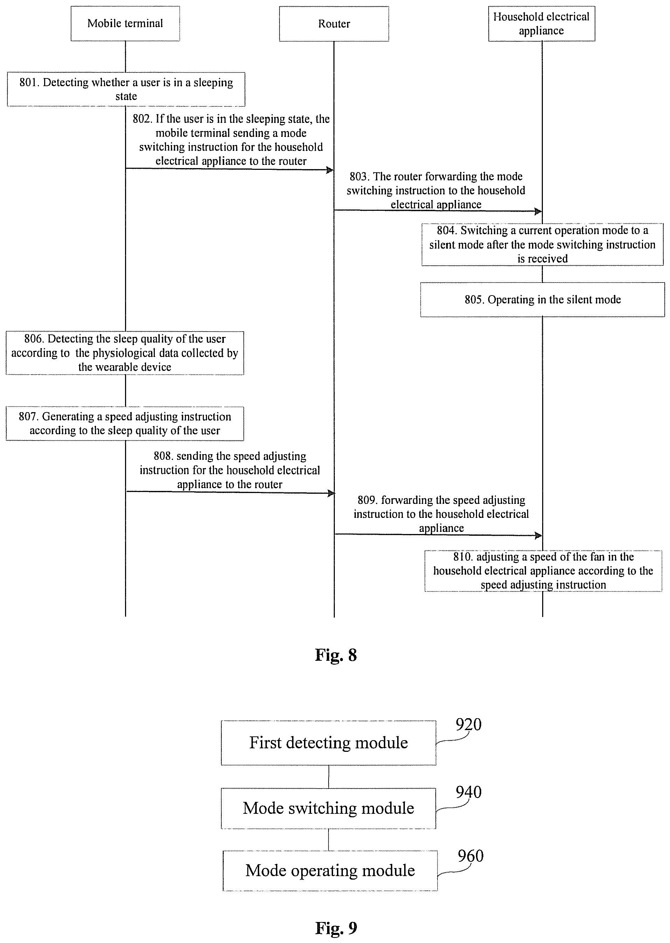

Fig.8 is a flow chart of a method for switching an operation mode according to further still another exemplary embodiment. In the present embodiment, the method for switching an operation mode is described as being applied, for example, in the implementation environment as shown in Fig.7. The method for switching an operation mode may include the following steps.

In step 801, the mobile terminal detects whether a user is in a sleeping state.

The mobile terminal detects whether the user is in the sleeping state, which may include three different implementations, respectively as follows.

As a first possible implementation, the step 801 may include but not limited to the following substeps.
1. The wearable device collects physiological data of the user, and the physiological data may include at least one of heartbeat, pulse and body movement.
2. The wearable device sends the collected physiological data of the user to the router, and the router forwards the physiological data to the mobile terminal.
3. The mobile terminal detects whether the user is in the sleeping state according to the physiological data.

When the heartbeat and pulse collected by the wearable device is below a normal value for a non-sleeping state and/or the corresponding body movement is below a predetermined frequency, the detection result of the mobile terminal is the user is in the sleeping state. When the heartbeat and pulse collected by the wearable device is at the normal value for a non-sleeping state and/or the corresponding body movement is above the predetermined frequency, the detection result of the mobile terminal is the user is in a non-sleeping state.

As a second possible implementation, the step 801 may include but not limited to the following substeps.
1. The mobile terminal acquires an operation state of a wireless access point.

The wireless access point may be a router supporting a Wi-Fi network at home. The wireless access point may be turned on or turned off manually to change the operation state of the wireless access point, or may be turned on during running time preset by the user, and turned off in non-running time, thereby to change the operation state of the wireless access point.

For example, the user presets 9:00-23:00 as the running time of the wireless access point, and 23:00-9:00 the next day as the non-running time of the wireless access point. When it is 9:00-23:00, the mobile terminal acquires that the wireless access point is in the running state, and when it is 23:00-9:00 the next day, the mobile terminal acquires that the wireless access point is in the non-running state.
2. The mobile terminal detects whether the user is in the sleeping state according to the operation state of the wireless access point.

When the wireless access point is in the running state, the detection result of the mobile terminal is the user is in the non-sleeping state; and when the wireless access point is in the non-running state, the detection result of the mobile terminal is the user is in the sleeping state.

As a third possible implementation, the step 801 may include but not limited to the following substeps.
1. The household electrical appliance acquires a noise value of a current environment, sends the noise value of the current environment to the router, and the router forwards the noise value of the current environment to the mobile terminal.

The household electrical appliance acquires the noise value of the current environment through a built-in noise meter or microphone, sends the noise value of the current environment to the router, and the router forwards the noise value of the current environment to the mobile terminal.
2. The mobile terminal detects whether the user is in the sleeping state according to the noise value of the current environment.

When the noise value of the current environment is larger than a bearable noise value for the sleeping state, the detection result of the mobile terminal is the user is in the non-sleeping state; and when noise value of the current environment is smaller than the bearable noise value for the sleeping state, the detection result of the mobile terminal is the user is in the sleeping state.

It should be noted that, in practical implementation of the present step, whether the user is in the sleeping state may be detected through one of the implementations; or whether the user is in the sleeping state may be detected through combination of any two of the implementations; or whether the user is in the sleeping state may be detected through combination of the three implementations. Wherein, reliability of the detection result through the combination of the three implementations is higher than reliability of the detection result through the combination of any two of the implementations, and the reliability of the detection result through the combination of any two of the implementations is higher than reliability of the detection result through one implementation.

It should be further noted that, in the combination of any two of the implementations or the combination of the three implementations, a weight may be assigned to each implementation of the two or three implementations according to a certain proportion of weights, and the detection result of the combined implementation may be calculated by taking all the weights into consideration.

In step 802, if the user is in the sleeping state, the mobile terminal sends a mode switching instruction for the household electrical appliance to the router.

In step 803, the router forwards the mode switching instruction to the household electrical appliance.

In step 804, the household electrical appliance switches a current operation mode to a silent mode after it receives the mode switching instruction.

In step 805, the household electrical appliance operates in the silent mode.

When the household electrical appliance is provided with a muffler, for example it is an air purifier provided with a muffler, the air purifier enables the muffler. The muffler may be a muffler layer or a muffler component for reducing noise which is disposed in an air flow passage or in an air intake system or an air exhaust system of an aerodynamic device (a device with a loud noise at an exhaust port, such as a fan or a blower).

When the household electrical appliance is further provided with a fan, the method may optionally include the following steps: in step 806, the mobile terminal detects the sleep quality of the user according to the physiological data collected by the wearable device.

When the user is in the sleep state, the sleep quality of the user may be divided into first sleep quality and second sleep quality. The first sleep quality may also be referred to as a "light sleep state", the second sleep quality may also be referred to as a "deep sleep state", and the sleep effect of the first sleep quality is poorer than that of the second sleep quality.

If the physiological data is consistent with physiological data of the first sleep quality, the detection result of the mobile terminal is the sleep quality of the user is in the first sleep quality. If the physiological data is consistent with physiological data of the second sleep quality, the detection result of the mobile terminal is the sleep quality of the user is in the second sleep quality.

In step 807, the mobile terminal generates a speed adjusting instruction according to the sleep quality of the user.

When the sleep quality of the user is in the first sleep quality, the mobile terminal generates a first speed adjusting instruction; and when the sleep quality of the user is in the second sleep quality, the mobile terminal generates a second speed adjusting instruction. Wherein, since the sleep effect of the first sleep quality is poorer than that of the second sleep quality, the first speed is smaller than the second speed.

In step 808, the mobile terminal sends the speed adjusting instruction for the household electrical appliance to the router.

The mobile terminal sends the speed adjusting instruction for the household electrical appliance to the router. In the present embodiment, for example, the first sleep quality corresponds to the first speed, and the second sleep quality corresponds to the second speed. The speed adjusting instruction may be the first speed adjusting instruction, or may also be the second speed adjusting instruction. In practical implementation, the speed adjusting instruction may include at least two types of speed adjusting instructions. The speed for the speed adjusting instruction may be preset by the user, or may be preset by maintenance personnel of the mobile terminal.

In step 809, the router forwards the speed adjusting instruction to the household electrical appliance.

In step 810, the household electrical appliance adjusts a speed of the fan in the household electrical appliance according to the speed adjusting instruction.

When the first speed adjusting instruction is received, the household electrical appliance adjusts the speed of the fan to the first speed; and when the second speed adjusting instruction is received, the household electrical appliance adjusts the speed of the fan to the second speed. Wherein, since the sleep effect of the first sleep quality is poorer than that of the second sleep quality, the first speed is smaller than the second speed.

The above steps 808 and 810 have another alternative implementation.

The mobile terminal adjusts the speed of the fan in the household electrical appliance according to the sleep quality of the user and current air quality.

The mobile terminal detects the sleep quality of the user according to the physiological data collected by the wearable device; the household electrical appliance detects the current air quality, and sends the current air quality to the router; the router forwards the current air quality to the mobile terminal; the mobile terminal generates the speed adjusting instruction according to the sleep quality of the user and the current air quality; the mobile terminal sends the speed adjusting instruction for the household electrical appliance to the router; the router forwards the speed adjusting instruction to the household electrical appliance; and the household electrical appliance adjusts the speed of the fan in the household electrical appliance according to the speed adjusting instruction.

That is, the mobile terminal may take both of the sleep quality of the user and the current air quality into consideration to control the speed of the fan. The speed of the fan after the adjustment positively correlates to the sleep quality, and the speed of the fan after the adjustment negatively correlates to the current air quality.

Assuming that the current air quality may be divided into first air quality, second air quality and third air quality. Wherein, the first air quality is better than the second air quality, and the second air quality is better than the third air quality.

When the sleep quality of the user is in the first sleep quality, and the current air quality is in the first air quality, the mobile terminal sends the first speed adjusting instruction for the household electrical appliance to the router, the router forwards the first speed adjusting instruction to the household electrical appliance, and the household electrical appliance adjusts the speed of the fan to the first speed according to the first speed adjusting instruction;
when the sleep quality of the user is in the second sleep quality, and the current air quality is in the first air quality, the mobile terminal sends the second speed adjusting instruction for the household electrical appliance to the router, the router forwards the second speed adjusting instruction to the household electrical appliance, and the household electrical appliance adjusts the speed of the fan to the second speed according to the second speed adjusting instruction;
when the sleep quality of the user is in the first sleep quality, and the current air quality is in the second air quality, the mobile terminal sends the third speed adjusting instruction for the household electrical appliance to the router, the router forwards the third speed adjusting instruction to the household electrical appliance, and the household electrical appliance adjusts the speed of the fan to the third speed according to the third speed adjusting instruction;
when the sleep quality of the user is in the second sleep quality, and the current air quality is in the second air quality, the mobile terminal sends the fourth speed adjusting instruction for the household electrical appliance to the router, the router forwards the fourth speed adjusting instruction to the household electrical appliance, and the household electrical appliance adjusts the speed of the fan to the fourth speed according to the fourth speed adjusting instruction;
when the sleep quality of the user is in the first sleep quality, and the current air quality is in the third air quality, the mobile terminal sends the fifth speed adjusting instruction for the household electrical appliance to the router, the router forwards the fifth speed adjusting instruction to the household electrical appliance, and the household electrical appliance adjusts the speed of the fan to the fifth speed according to the fifth speed adjusting instruction; and
when the sleep quality of the user is in the second sleep quality, and the current air quality is in the third air quality, the mobile terminal sends the sixth speed adjusting instruction for the household electrical appliance to the router, the router forwards the sixth speed adjusting instruction to the household electrical appliance, and the household electrical appliance adjusts the speed of the fan to the sixth speed according to the sixth speed adjusting instruction.

Wherein, since the sleep effect of the first sleep quality is poorer than that of the second sleep quality, the first air quality is better than the second air quality, and the second air quality is better than the third air quality, then, the first speed is smaller than the second speed, the second speed is smaller than the third speed, the third speed is smaller than the fourth speed, the fourth speed is smaller than the fifth speed, and the fifth speed is smaller than the sixth speed.

It should be further noted that, the method for switching an operation mode provided by the present embodiment may also include:
the mobile terminal establishes in advance a binding relationship with the wearable device and the household electrical appliance.

The mobile terminal establishes in advance a binding relationship among the mobile terminal, the wearable device and the household electrical appliance according to an identification of the mobile terminal, an identification of the wearable device and an identification of the household electrical appliance. The household electrical appliance only receives controlling instruction sent by the mobile terminal which has an established binding relationship therewith.

In the method for switching an operation mode provided by the present embodiment, it is detected whether a user is in a sleeping state; if the user is in the sleeping state, a mode switching instruction is sent to the household electrical appliance; the household electrical appliance being configured to switch a current operation mode to a silent mode, and operate in the silent mode. Accordingly, it may be solved the problem that a process of adjusting the mobile terminal to the silent mode through a remote controller or in a manual manner is still a manual control which involves troublesome operations, and it may be achieved an effect that the process for switching an operation mode may be simplified, and the silent mode may be operated through automatic control.

In addition, in the method for switching an operation mode provided by the present embodiment, the sleep quality of the user is detected according to the physiological data collected by the wearable device in the silent mode; and the speed of the fan in the mobile terminal is adjusted according to the sleep quality of the user. Accordingly, it may be achieved an effect that the speed of the fan may be automatically adjusted according to the sleep quality and the noise may be reduced.

The following are device embodiments of the present disclosure, which may be configured to perform the method embodiments of the present disclosure. For details that are not disclosed in the device embodiments of the present disclosure, reference may be made to the method embodiments of the present disclosure.

Fig.9 is a block diagram of a device for switching an operation mode according to an exemplary embodiment. The device for switching an operation mode may be implemented as a part or a whole of the household electrical appliance in the implementation environment shown in Fig.1 in a form of software, hardware or combination of the both. The device for switching an operation mode may include:
a first detecting module 920, configured to detect whether a user is in a sleeping state;
a mode switching module 940, configured to, if the user is in the sleeping state, switch a current operation mode to a silent mode; and
a mode operating module 960, configured to operate in the silent mode.

In the device for switching an operation mode provided by the present embodiment, it is detected whether a user is in a sleeping state; if the user is in the sleeping state, a current operation mode is switched to a silent mode; and then it is operated in the silent mode. Accordingly, it may be solved the problem that a process of adjusting the household electrical appliance to the silent mode through a remote controller or in a manual manner is still a manual control which involves troublesome operations, and it may be achieved an effect that the process for switching an operation mode may be simplified, and the silent mode may be operated through automatic control.

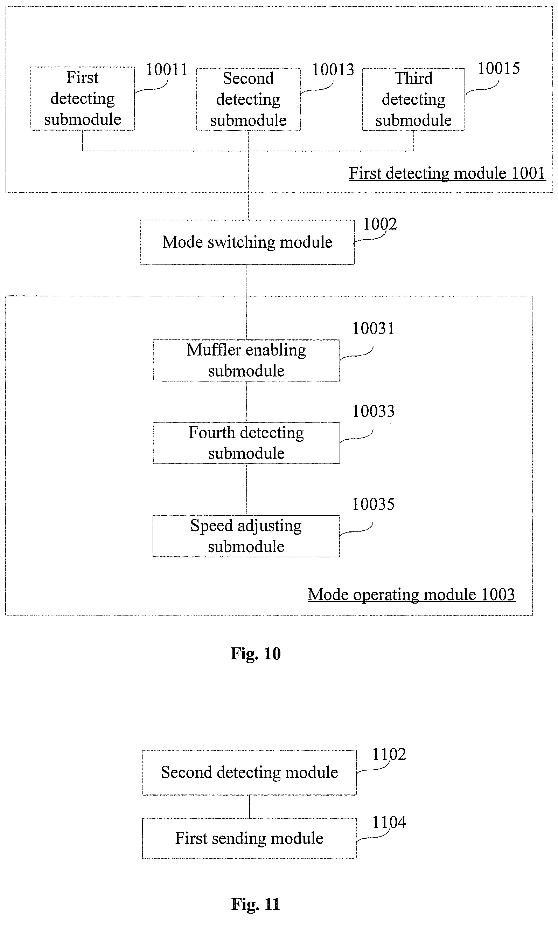

Fig.10 is a block diagram of a device for switching an operation mode according to another exemplary embodiment. The device for switching an operation mode may be implemented as a part or a whole of the household electrical appliance in the implementation environment shown in Fig.1 in a form of software, hardware or combination of the both. The device for switching an operation mode may include:
a first detecting module 1001, configured to detect whether a user is in a sleeping state;
a mode switching module 1002, configured to, if the user is in the sleeping state, switch a current operation mode to a silent mode; and
a mode operating module 1003, configured to operate in the silent mode.

Optionally, the first detecting module 1001 includes:
a first detecting submodule 10011, configured to acquire physiological data of the user which is collected by a wearable device, and detect whether the user is in the sleeping state according to the physiological data;
   and/or,
a second detecting submodule 10013, configured to acquire an operation state of a wireless access point, and detect whether the user is in the sleeping state according to the operation state of the wireless access point;
   and/or,
a third detecting submodule 10015, configured to acquire a noise value of a current environment, and detect whether the user is in the sleeping state according to the noise value of the current environment.

Optionally, the mode operating module 1003 includes:
a muffler enabling submodule 10031, configured to, when the household electrical appliance is provided with a muffler, enable the muffler.

Optionally, the mode operating module 1003 includes:
a fourth detecting submodule 10033, configured to detect sleep quality of the user according to the physiological data collected by the wearable device; and
a speed adjusting submodule 10035, configured to adjust a speed of the fan in the household electrical appliance according to the sleep quality of the user.

Optionally, the speed adjusting submodule 10035 is configured to adjust the speed of the fan according to the sleep quality of the user and current air quality.

In the device for switching an operation mode provided by the present embodiment, it is detected whether a user is in a sleeping state; if the user is in the sleeping state, a current operation mode is switched to a silent mode; and then it is operated in the silent mode. Accordingly, it may be solved the problem that a process of adjusting the household electrical appliance to the silent mode through a remote controller or in a manual manner is still a manual control which involves troublesome operations, and it may be achieved an effect that the process for switching an operation mode may be simplified, and the silent mode may be operated through automatic control.

In addition, in the device for switching an operation mode provided by the present embodiment, the sleep quality of the user is detected according to the physiological data collected by the wearable device in the silent mode; and the speed of the fan in the household electrical appliance is adjusted according to the sleep quality of the user. Accordingly, it may be achieved an effect that the speed of the fan may be automatically adjusted according to the sleep quality and the noise may be reduced.

Fig.11 is a block diagram of a device for switching an operation mode according to still another exemplary embodiment. The device for switching an operation mode may be implemented as a part or a whole of the router in the implementation environment shown in Fig.4 or of the mobile terminal in the implementation environment shown in Fig.7 in a form of software, hardware or combination of the both. The device for switching an operation mode may include:
a second detecting module 1102, configured to detect whether a user is in a sleeping state; and
a sending module 1104, configured to, when the user is in the sleeping state, send a mode switching instruction to the household electrical appliance; the household electrical appliance being configured to switch a current operation mode to a silent mode according to the mode switching instruction, and operate in the silent mode.

In the device for switching an operation mode provided by the present embodiment, it is detected whether a user is in a sleeping state; and if the user is in the sleeping state, a mode switching instruction is sent to the household electrical appliance; the household electrical appliance being configured to switch a current operation mode to a silent mode according to the mode switching instruction, and operate in the silent mode. Accordingly, it may be solved the problem that a process of adjusting the household electrical appliance to the silent mode through a remote controller or in a manual manner is still a manual control which involves troublesome operations, and it may be achieved an effect that the process for switching an operation mode may be simplified, and the silent mode may be operated through automatic control.

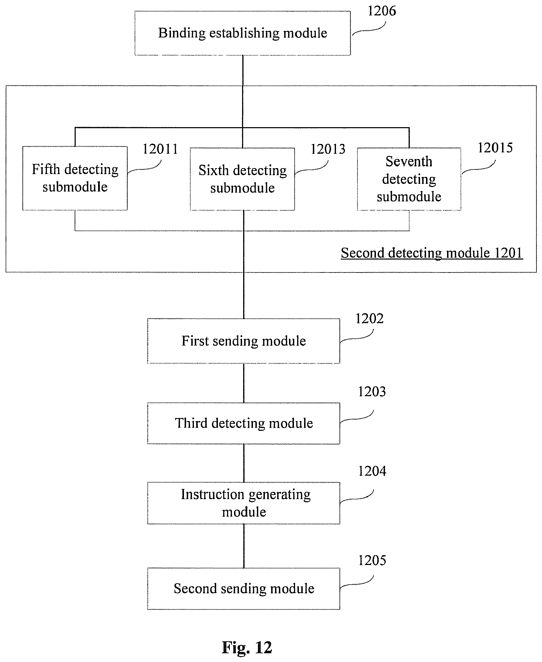

Fig.12 is a block diagram of a device for switching an operation mode according to further still another exemplary embodiment. The device for switching an operation mode may be implemented as a part or a whole of the router in the implementation environment shown in Fig.4 or of the mobile terminal in the implementation environment shown in Fig.7 in a form of software, hardware or combination of the both. The device for switching an operation mode may include:
a second detecting module 1201, configured to detect whether a user is in a sleeping state; and
a first sending module 1202, configured to, when the user is in the sleeping state, send a mode switching instruction to the household electrical appliance; the household electrical appliance being configured to switch a current operation mode to a silent mode according to the mode switching instruction, and operate in the silent mode.

In exemplary embodiments, the device 1300 may be implemented with one or more application specific integrated circuits (ASICs), digital signal processors (DSPs), digital signal processing devices (DSPDs), programmable logic devices (PLDs), field programmable gate arrays (FPGAs), controllers, micro-controllers, microprocessors, or other electronic components, for performing the method for switching an operation mode as shown in Fig.2 or 3.

In exemplary embodiments, there is also provided a non-transitory computer-readable storage medium including instructions, such as included in the memory 1304, executable by the processor 1320 in the device 1300, for performing the above-described methods. For example, the non-transitory computer-readable storage medium may be a ROM, a RAM, a CD-ROM, a magnetic tape, a floppy disc, an optical data storage device, and the like.

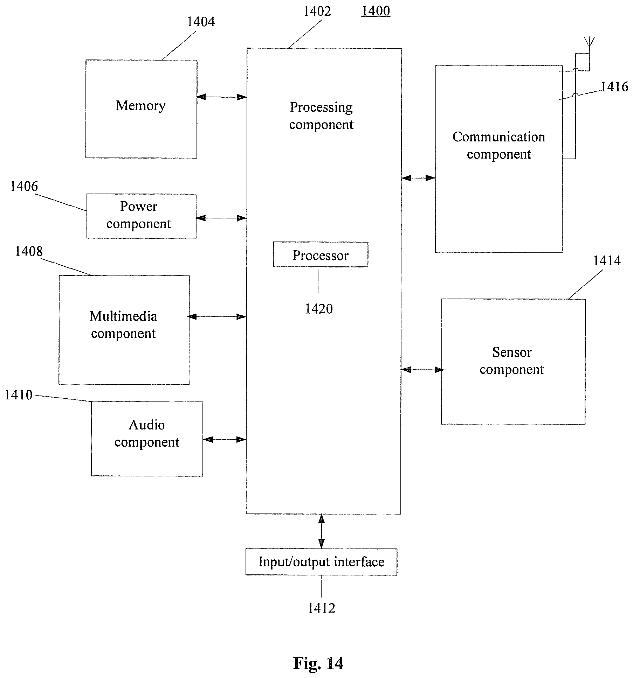

Fig.14 is a block diagram of a device 1400 for switching an operation mode according to another exemplary embodiment. The device is for example used in the router in the implementation environment shown in Fig.4 or in the mobile terminal in the implementation environment shown in Fig.7. For example, the device 1400 may be a mobile phone, a computer, a digital broadcast terminal, a messaging device, a gaming console, a tablet, a medical device, exercise equipment, a personal digital assistant, and the like.

Referring to Fig.14, the device 1400 may include one or more of the following components: a processing component 1402, a memory 1404, a power component 1406, a multimedia component 1408, an audio component 1410, an input/output (I/O) interface 1412, a sensor component 1414, and a communication component 1416.

The processing component 1402 typically controls overall operations of the device 1400, such as the operations associated with display, telephone calls, data communications, camera operations, and recording operations. The processing component 1402 may include one or more processors 1420 to execute instructions to perform all or part of the steps in the above described methods. Moreover, the processing component 1402 may include one or more modules which facilitate the interaction between the processing component 1402 and other components. For instance, the processing component 1402 may include a multimedia module to facilitate the interaction between the multimedia component 1408 and the processing component 1402.

The memory 1404 is configured to store various types of data to support the operation of the device 1400. Examples of such data include instructions for any applications or methods operated on the device 1400, contact data, phonebook data, messages, pictures, video, etc. The memory 1404 may be implemented using any type of volatile or non-volatile memory devices, or a combination thereof, such as a static random access memory (SRAM), an electrically erasable programmable read-only memory (EEPROM), an erasable programmable read-only memory (EPROM), a programmable read-only memory (PROM), a read-only memory (ROM), a magnetic memory, a flash memory, a magnetic or optical disk.

The power component 1406 provides power to various components of the device 1400. The power component 1406 may include a power management system, one or more power sources, and any other components associated with the generation, management, and distribution of power in the device 1400.

The multimedia component 1408 includes a screen providing an output interface between the device 1400 and the user. In some embodiments, the screen may include a liquid crystal display (LCD) and a touch panel (TP). If the screen includes the touch panel, the screen may be implemented as a touch screen to receive input signals from the user. The touch panel includes one or more touch sensors to sense touches, swipes, and gestures on the touch panel. The touch sensors may not only sense a boundary of a touch or swipe action, but also sense a period of time and a pressure associated with the touch or swipe action. In some embodiments, the multimedia component 1408 includes a front camera and/or a rear camera. The front camera and the rear camera may receive an external multimedia datum while the device 1400 is in an operation mode, such as a photographing mode or a video mode. Each of the front camera and the rear camera may be a fixed optical lens system or have focus and optical zoom capability.

The audio component 1410 is configured to output and/or input audio signals. For example, the audio component 1410 includes a microphone ("MIC") configured to receive an external audio signal when the device 1400 is in an operation mode, such as a call mode, a recording mode, and a voice recognition mode. The received audio signal may be further stored in the memory 1404 or transmitted via the communication component 1416. In some embodiments, the audio component 1410 further includes a speaker to output audio signals.

The I/O interface 1412 provides an interface between the processing component 1402 and peripheral interface modules, such as a keyboard, a click wheel, buttons, and the like. The buttons may include, but are not limited to, a home button, a volume button, a starting button, and a locking button.

The sensor component 1414 includes one or more sensors to provide status assessments of various aspects of the device 1400. For instance, the sensor component 1414 may detect an open/closed status of the device 1400, relative positioning of components, e.g., the display and the keypad, of the device 1400, a change in position of the device 1400 or a component of the device 1400, a presence or absence of user contact with the device 1400, an orientation or an acceleration/deceleration of the device 1400, and a change in temperature of the device 1400. The sensor component 1414 may include a proximity sensor configured to detect the presence of nearby objects without any physical contact. The sensor component 1414 may also include a light sensor, such as a CMOS or CCD image sensor, for use in imaging applications. In some embodiments, the sensor component 1414 may also include an accelerometer sensor, a gyroscope sensor, a magnetic sensor, a pressure sensor, or a temperature sensor.

The communication component 1416 is configured to facilitate communication, wired or wirelessly, between the device 1400 and other devices. The device 1400 can access a wireless network based on a communication standard, such as WiFi, 2G, or 3G, or a combination thereof. In one exemplary embodiment, the communication component 1416 receives a broadcast signal or broadcast associated information from an external broadcast management system via a broadcast channel. In one exemplary embodiment, the communication component 1416 further includes a near field communication (NFC) module to facilitate short-range communications. For example, the NFC module may be implemented based on a radio frequency identification (RFID) technology, an infrared data association (IrDA) technology, an ultra-wideband (UWB) technology, a Bluetooth (BT) technology, and other technologies.

In exemplary embodiments, the device 1400 may be implemented with one or more application specific integrated circuits (ASICs), digital signal processors (DSPs), digital signal processing devices (DSPDs), programmable logic devices (PLDs), field programmable gate arrays (FPGAs), controllers, micro-controllers, microprocessors, or other electronic components, for performing the method for switching an operation mode as shown in Fig.5, 6 or 8.

In exemplary embodiments, there is also provided a non-transitory computer-readable storage medium including instructions, such as included in the memory 1404, executable by the processor 1420 in the device 1400, for performing the above-described methods. For example, the non-transitory computer-readable storage medium may be a ROM, a RAM, a CD-ROM, a magnetic tape, a floppy disc, an optical data storage device, and the like.

It will be appreciated that the present invention is not limited to the exact construction that has been described above and illustrated in the accompanying drawings, and that various modifications and changes can be made without departing from the scope thereof. It is intended that the scope of the invention only be limited by the appended claims.

## Claims

1. A method for switching an operation mode, the method being applied in a household electrical appliance (140) and comprising:
detecting whether a user is
in a sleeping state (202, 301), and if the user is in the sleeping state:
- detecting sleep quality of the user according to physiological data collected by a wearable device;
- adjusting a speed of a fan in said household electrical appliance according to the sleep quality of the user and current air quality; **characterized in that**
the step of detecting whether a user is in a sleeping state comprises acquiring an operation state of a wireless access point, and detecting whether the user is in the sleeping state according to the operation state of the wireless access point.

2. A device for switching an operation mode, whereby the device is applied in a controlling device coupled to a household electrical appliance, and comprises:
a detecting module (1102), configured to detect whether a user is in a sleeping state; and
a detecting submodule (12011), configured to acquire physiological data of the user which is collected by a wearable device, and detect whether the user is in the sleeping state according to the physiological data;
a detection module (1203), configured to detect sleep quality of the user according to the physiological data collected by the wearable device;
an instruction generating module (1204) configured to generate a speed adjusting instruction according to the sleep quality of the user and current air quality;
a sending module (1205) configured to send a speed adjusting instruction to the household electrical appliance; the household electrical appliance being configured to adjust a speed of a fan in the household electrical appliance according to the speed adjusting instruction; **characterized in that**
the detecting module comprises: a detecting submodule (12013), configured to acquire an operation state of a wireless access point, and detect whether the user is in the sleeping state according to the operation state of the wireless access point.

3. The device according to claim 2, **characterized in that** the device further comprises:
a binding establishing module (1206), configured to establish in advance a binding relationship with the wearable device and the household electrical appliance.

4. A device for switching an operation mode, whereby the device is applied in a household electrical appliance, and comprises:
a processor (1320); and
a memory (1304) for storing instructions executable by the processor (1320);
wherein the processor is configured to perform:
detecting whether a user is in a sleeping state and if the user is in the sleeping state :
- detecting sleep quality of the user according to physiological data collected by a wearable device; the device being **characterized in that** the processor is configured to perform:
- adjusting a speed of a fan in said household electrical appliance according to the sleep quality of the user and current air quality; **characterized in that**
the detecting whether a user is in a sleeping state comprises acquiring an operation state of a wireless access point, and detecting whether the user is in the sleeping state according to the operation state of the wireless access point.

5. A computer program including instructions for executing the steps of a method for switching an operation mode according to claim 1 when said program is executed by a computer.

6. A recording medium readable by a computer and having recorded thereon a computer program including instructions for executing the steps of a method for switching an operation mode according to claim 1 when said program is executed by a computer.

## Patentansprüche

1. Verfahren zum Umschalten eines Betriebsmodus, wobei das Verfahren in einem elektrischen Haushaltsgerät (140) angewendet wird und umfasst:
Erkennen, ob ein Benutzer in einem schlafenden Zustand (202, 301) ist, und, wenn der Benutzer im schlafenden Zustand ist:
- Erkennen von Schlafqualität des Benutzers gemäß physiologischen Daten, die durch ein tragbares Gerät gesammelt werden,
- Anpassen einer Geschwindigkeit eines Gebläses im elektrischen Haushaltsgerät gemäß der Schlafqualität des Benutzers und gegenwärtiger Luftqualität, **dadurch gekennzeichnet, dass**
der Schritt des Erkennens, ob ein Benutzer im schlafenden Zustand ist, das Erfassen eines Betriebszustands eines drahtlosen Zugangspunkts und das Erkennen, ob der Benutzer im schlafenden Zustand ist, gemäß dem Betriebszustand des drahtlosen Zugangspunkts umfasst.

2. Vorrichtung zum Umschalten eines Betriebsmodus, wobei die Vorrichtung in einer Steuervorrichtung angewendet ist, die an ein elektrisches Haushaltsgerät gekoppelt ist, und umfasst:
ein Erkennungsmodul (1102), das zum Erkennen konfiguriert ist, ob ein Benutzer in einem schlafenden Zustand ist, und
ein Erkennungsnebenmodul (12011), das zum Erfassen von physiologischen Daten des Benutzers, die durch ein tragbares Gerät gesammelt werden, und zum Erkennen, ob der Benutzer im schlafenden Zustand ist, gemäß den physiologischen Daten konfiguriert ist,
ein Erkennungsmodul (1203), das zum Erkennen von Schlafqualität des Benutzers gemäß den physiologischen Daten, die durch das tragbare Gerät gesammelt werden, konfiguriert ist,
ein Befehlserzeugungsmodul (1204), das zum Erzeugen eines Geschwindigkeitseinstellungsbefehls gemäß der Schlafqualität des Benutzers und gegenwärtiger Luftqualität konfiguriert ist,
ein Sendemodul (1205), das zum Senden eines Geschwindigkeitseinstellungsbefehls an das elektrische Haushaltsgerät konfiguriert ist; wobei das elektrische Haushaltsgerät zum Einstellen einer Geschwindigkeit eines Gebläses im elektrischen Haushaltsgerät gemäß dem Geschwindigkeitseinstellungsbefehl konfiguriert ist, **dadurch gekennzeichnet, dass**
das Erkennungsmodul umfasst: ein Erkennungsnebenmodul (12013), das zum Erfassen eines Betriebszustands eines drahtlosen Zugangspunkts und zum Erkennen, ob der Benutzer im schlafenden Zustand ist, gemäß dem Betriebszustand des drahtlosen Zugangspunkts konfiguriert ist.

3. Vorrichtung nach Anspruch 2, **dadurch gekennzeichnet, dass** die Vorrichtung ferner umfasst:
ein Bindungsherstellungmodul (1206), das zum Herstellen einer Bindungsbeziehung mit dem tragbaren Gerät und dem elektrischen Haushaltsgerät im Voraus konfiguriert ist.

4. Vorrichtung zum Umschalten eines Betriebsmodus, wobei die Vorrichtung in einem elektrischen Haushaltsgerät angewendet ist und umfasst:
einen Prozessor (1320) und
einen Speicher (1304) zum Speichern von Befehlen, die durch den Prozessor (1320) ausführbar sind,
wobei der Prozessor dazu konfiguriert ist, auszuführen:
Erkennen, ob ein Benutzer in einem schlafenden Zustand ist, und, wenn der Benutzer im schlafenden Zustand ist:
- Erkennen von Schlafqualität des Benutzers gemäß physiologischen Daten, die durch ein tragbares Gerät gesammelt werden, wobei die Vorrichtung **dadurch gekennzeichnet ist, dass** der Prozessor dazu konfiguriert ist, auszuführen:
- Anpassen einer Geschwindigkeit eines Gebläses im elektrischen Haushaltsgerät gemäß der Schlafqualität des Benutzers und gegenwärtiger Luftqualität, **dadurch gekennzeichnet, dass**
das Erkennen, ob ein Benutzer im schlafenden Zustand ist, das Erfassen eines Betriebszustands eines drahtlosen Zugangspunkts und das Erkennen, ob der Benutzer im schlafenden Zustand ist, gemäß dem Betriebszustand des drahtlosen Zugangspunkts umfasst.

5. Computerprogramm, das Befehle zum Ausführen der Schritte eines Verfahrens zum Umschalten eines Betriebszustands gemäß Anspruch 1 enthält, wenn das Programm durch einen Rechner ausgeführt wird.

6. Aufzeichnungsmedium, das durch einen Rechner lesbar ist und darauf ein Computerprogramm aufgezeichnet aufweist, das Befehle zum Ausführen der Schritte eines Verfahrens zum Umschalten eines Betriebszustands gemäß Anspruch 1 enthält, wenn das Programm durch einen Rechner ausgeführt wird.

## Revendications

1. Procédé d'activation d'un mode de fonctionnement, le procédé étant appliqué à un appareil électroménager (140) et comprenant :
la détection du fait qu'un utilisateur soit en train de dormir ou non (202, 301) et, si l'utilisateur est en train de dormir :
- la détection de la qualité de sommeil de l'utilisateur selon des données physiologiques collectées par un dispositif portable ;
- l'ajustement d'une vitesse d'un ventilateur dans ledit appareil électroménager selon la qualité de sommeil de l'utilisateur et la qualité actuelle de l'air ; **caractérisé en ce que**
l'étape de détection du fait qu'un utilisateur soit en train de dormir ou non comprend l'acquisition d'un état de fonctionnement d'un point d'accès sans fil, et la détection du fait que l'utilisateur soit en train de dormir ou non selon l'état de fonctionnement du point d'accès sans fil.

2. Dispositif d'activation d'un mode de fonctionnement, dans lequel le dispositif est appliqué dans un dispositif de contrôle relié à un appareil électroménager, et comprend :
un module de détection (1102), configuré pour détecter si un utilisateur est en train de dormir ou non ; et
un sous-module de détection (12011), configuré pour acquérir des données physiologiques de l'utilisateur qui sont collectées par un dispositif portable, et pour détecter si l'utilisateur est en train de dormir ou non selon les données physiologiques ;
un module de détection (1203), configuré pour détecter la qualité de sommeil de l'utilisateur selon les données physiologiques collectées par le dispositif portable ;
un module de génération d'instructions (1204) configuré pour générer une instruction d'ajustement de vitesse selon la qualité de sommeil de l'utilisateur et la qualité actuelle de l'air ;
un module d'envoi (1205) configuré pour envoyer une instruction d'ajustement de vitesse à l'appareil électroménager ; l'appareil électroménager étant configuré pour ajuster une vitesse d'un ventilateur dans l'appareil électroménager selon l'instruction d'ajustement de vitesse ; **caractérisé en ce que**
le module de détection comprend : un sous-module de détection (12013), configuré pour acquérir un état de fonctionnement d'un point d'accès sans fil, et pour détecter si l'utilisateur est en train de dormir ou non selon l'état de fonctionnement du point d'accès sans fil.

3. Dispositif selon la revendication 2, **caractérisé en ce que** le dispositif comprend en outré :
un module d'établissement de liaison (1206), configuré pour établir à l'avance une liaison avec le dispositif portable et l'appareil électroménager.

4. Dispositif d'activation d'un mode de fonctionnement, moyennant quoi le dispositif est appliqué dans un appareil électroménager, et comprend :
un processeur (1320) ; et
une mémoire (1304) destinée à stocker des instructions exécutables par le processeur (1320) ;
dans lequel le processeur est configuré pour :
détecter si un utilisateur est en train de dormir ou non et, si l'utilisateur est en train de dormir :
- détecter la qualité de sommeil de l'utilisateur selon des données physiologiques collectées par un dispositif portable ; le dispositif étant **caractérisé en ce que** le processeur est configuré pour :
- ajuster une vitesse d'un ventilateur dans ledit appareil électroménager selon la qualité de sommeil de l'utilisateur et la qualité actuelle de l'air ; **caractérisé en ce que**
la détection du fait qu'un utilisateur soit en train de dormir ou non comprend l'acquisition d'un état de fonctionnement d'un point d'accès sans fil, et la détection du fait que l'utilisateur soit en train de dormir ou non selon l'état de fonctionnement du point d'accès sans fil.

5. Programme informatique comprenant des instructions destinées à exécuter les étapes d'un procédé d'activation d'un mode de fonctionnement selon la revendication 1 lorsque ledit programme est exécuté par un ordinateur.

6. Support d'enregistrement lisible par un ordinateur et contenant un programme informatique comprenant des instructions destinées à exécuter les étapes d'un procédé d'activation d'un mode de fonctionnement selon la revendication 1 lorsque ledit programme est exécuté par un ordinateur.
